# EUROPEAN PATENT SPECIFICATION

(11) **EP 1 210 018 B1**
(45) Date of publication and mention of the grant of the patent: **04.02.2004**
(21) Application number: 00958795.7
(22) Date of filing: 06.09.2000
(51) Int. Cl.: A61B 17/58

(54) **A TAMP ASSEMBLY**
KNOCHENTRANSPLANTATVERDICHTER
ENSEMBLE DE TASSAGE

(30) Priority: 08.09.1999 GB 9921099
(43) Date of publication of application: 05.06.2002
(73) Proprietor: DEPUY INTERNATIONAL LIMITED, Beeston, Leeds LS11 8DT (GB)
(72) Inventor: FOLEY, Frank, West Byfleet, Surrey KT14 7AL (GB); LATIMORE, Rob, Limington SO41 9FR (GB)
(74) Representative: Belcher, Simon James
(86) International application number: PCT/GB2000/003410
(87) International publication number: WO 2001/017444

(56) References cited:
- EP-A- 0 888 752
- US-A- 5 385 566
- US-A- 5 665 121
- US-A- 5 683 395
- US-A- 5 910 172

## Description

This invention relates to a tamp assembly for use in preparing a bone for implantation of a component of a joint prosthesis.

Commonly used techniques for implantation of a joint prosthesis involve providing a filler material into the bone cavity before locating the prosthesis in the cavity. The filler material can help to minimise voids within the cavity around the prosthesis and can facilitate the formation of bonds between the prosthesis and the natural bone tissue. The filler material can be one which encourages the growth of natural bone tissue by which the prosthesis can be secured in place within the cavity; suitable materials include morcelised bone graft. The filler material might be a cement material which itself forms a bond to the prosthesis; commonly used bone cement materials include those based on acrylate materials.

Filler material that is located in the bone cavity needs to be packed densely within the cavity to minimise voids. It also needs to be shaped to match the configuration of the prosthesis. It is known to shape material within a bone cavity by means of a tamp which is forced into the cavity to compress the material so that it adopts the configuration that is desired to suit the prosthesis that is to be implanted. A surgeon might use a number of tamps in sequence, whose configurations tend closer to that of the prosthesis. This has been found to be an advantageous technique for making the filler material dense and also match the configuration of the prosthesis component to be implanted. For example, the configuration of a set of tamps for a prosthesis that is tapered gradually towards its tip might change from being tapered sharply so that it is wide at its end facing the handle, to being tapered less sharply so that it is relatively narrow at its end facing the handle.

It is a disadvantage of known instrument sets of the kind described above which include a plurality of tamps that the sets include a large number of components. Indeed, frequently, the number of instruments is increased significantly because the surgeon might only select the prosthesis component for a particular patient during the course of surgery and instruments would have to be provided for each possible prosthesis component that might be used.

According to the present invention, it has been found that the inventory of instruments for preparing a bond cavity for implantation of a prosthesis can be reduced by use of a tamp assembly which comprises a handle, a first tamp part, and a plurality of second tamp parts, each of which can be located between the handle and the first tamp part. The second tamp parts have different configurations and can be used sequentially to shape material within the bone cavity.

Accordingly, in one aspect, the invention provides a tamp assembly for use in preparing a bone for implantation of a component of a joint prosthesis, comprising:
a. a handle,
b. a first tamp part located remote from the handle,
c. a plurality of second tamp parts, each of which can be located between the handle and the first tamp part, the cross-sectional configuration of the second tamp parts at their end which face towards and contact the first tamp part substantially matching the cross-sectional configuration of the first tamp part on its end which faces and contacts the second tamp part, each of the second tamp parts being flared outwardly from its end which faces towards the first tamp part towards the end which faces towards the handle with the shape of the flare varying between individual ones of the second tamp parts.

The assembly of the present invention has the advantage that it can present the surgeon effectively with a wide range of tamps of differing configurations without the need to produce and to maintain an inventory of components which includes several complete tamps. This is achieved according to the invention by providing a first tamp part which is used with several second tamp parts. Each of those second tamp parts together with the first tamp part defines the configuration for each of a number of tamps which can be used successively to pack filler material into a bone cavity prior to implantation of a joint prosthesis component.

The assembly of the invention can be used to prepare cavities in preparation for implantation of components of any of a range of joint prostheses. For example, the assembly can be used to prepare the femur or the tibia for implantation of a component of a knee joint prosthesis. It can be used to prepare bones for implantation of components of other joints such as a hip joint, a shoulder joint or an ankle joint.

The configurations of the second tamp parts will be selected according to the configuration of the prosthesis component that is to be implanted so as to prepare the cavity to match the tapered configuration of the prosthesis component. The second tamp part which is intended to be used last will have a configuration such that the configuration of the tamp as a whole matches closely the configuration of the prosthesis component. The configurations of the second tamp parts might therefore vary between a first part in the series which is tapered sharply so that it is wide at its end facing the handle, and a second part which is tapered less sharply so that it is relatively narrow at its end facing the handle.

Generally, the first tamp part has a cross-section at the end which faces towards the second tamp part which is substantially circular. The mating face of each of the second tamp parts will then preferably also be substantially circular. Preferably, the cross-section of the end of the second tamp parts which faces towards the handle is substantially elliptical. It will be understood that the cross-sectional shapes can vary from circular or elliptical in the strict geometric sense. For example, the cross-sectional shape might be flattened on one side of the tamp part. An elliptical cross-section at the end of the second tamp part which faces the handle will deviate from being circular by having major and minor axes: it might however be generally oval rather than elliptical in the strict geometric sense. When the second tamp parts are elliptical at the end which faces towards the handle, it is preferred that they differ from one another in the eccentricity of the elliptical cross-section at the end which faces the handle. Preferably, the eccentricity of the elliptical cross-section (defined as the ratio of the length of the major axis to the length of the minor axis) of at least one of the second tamp parts at the end which faces the handle is at least about 1.3, more preferably at least about 1.5, especially at least about 1.7. Preferably, the eccentricity of the elliptical cross-section of at least one of the second tamp parts at the end which faces the handle is not more than about 1.3, more preferably not more than about 1.2, especially not more than about 1.1.

The first tamp part will preferably have a cross-section which changes little along its length. It might be preferred for the first tamp part to be tapered inwardly towards the end that is remote from the handle. However, it will generally be preferred that the angle of the taper is small so that, apart from any rounding at the tip of the first tamp part, the cross-sectional shape of the first tamp part is generally similar at the end which faces the second tamp part to the cross-sectional shape towards the other end.

Preferably, the tamp assembly includes a shaft which extends between the handle and the first tamp part by which the handle and the first and second tamp parts are connected to one another. Preferably, the shaft is attached to one of the handle and the first tamp part, and can be separably connected to the other of the handle and the first tamp part. Preferably, each of the second tamp parts has a bore extending through it through which the shaft can extend. The use of a shaft which extends through the second tamp part provides a convenient and simple technique for assembly of the tamp. The formation of the connection between one of the handle and the first tamp part and a shaft on the other of the handle and the first tamp part means that the second tamp part does not need to have formed on it any formations by which the connection is made. This means that manufacture of the second tamp parts is kept simple. Preferably, the assembly includes features which ensure proper angular alignment of the tamp parts with one another. For example, the bore through the second tamp part and the shaft might each have non-circular cross-section, especially with a flattened face.

The connection between components of the tamp assembly can be made by means of conventional connector arrangements. Examples might include for example cooperating threads, a bayonet connection, a retractable tang which can fit into a recess (especially with the tang on the handle and the recess being formed in the shaft) etc. Preferably, the components are configured so that relative rotation between the components is inhibited. For example, when the second tamp part has a bore extending through it for a shaft, and assembly of the tamp involves connecting the shaft to the handle, the end of the shaft can have a non-circular cross section (for example a square cross-section) which is received in an opening in the handle with a corresponding cross-section.

Preferably, components of the tamp assembly are cannulated so that it can accommodate a guide rod. A guide rod can then be fitted into the medullary cavity within the bone, for example on a cement restrictor plug, and the tamp assembly moved within the cavity along the guide rod which controls the orientation of the tamp assembly while the filler material within the cavity is being compressed. When the assembly includes a shaft which extends through the second tamp part between the first tamp part and the handle, the cannula will extend through the shaft which will be hollow. When the tamp components are cannulated, it is preferred that the assembly of the invention includes a guide rod which can be received in the cannula. The location and fixation of a cement restrictor plug within the medullary cavity can be achieved using an instrument which includes a bearing sleeve, as disclosed in US-5387216.

Embodiments of the present invention will now be described by way of example with reference to the accompanying drawings, in which:
Figure 1 is a side view of a first tamp part of the assembly of the invention.
Figures 2 (a), (b) and (c) are views from above of a series of second tamp parts.
Figures 3 (a), (b) and (c) are side views of the second tamp parts shown in Figure 2.
Figure 4 is a side view of the assembly of the invention incorporating the first tamp part shown in Figure 1 and the second tamp part shown in Figure 2 (a).
Figure 5 is a side view, partially in section, of a patient's tibia which is being prepared for implantation of the tibial component of a knee joint using the tamp assembly of the invention.

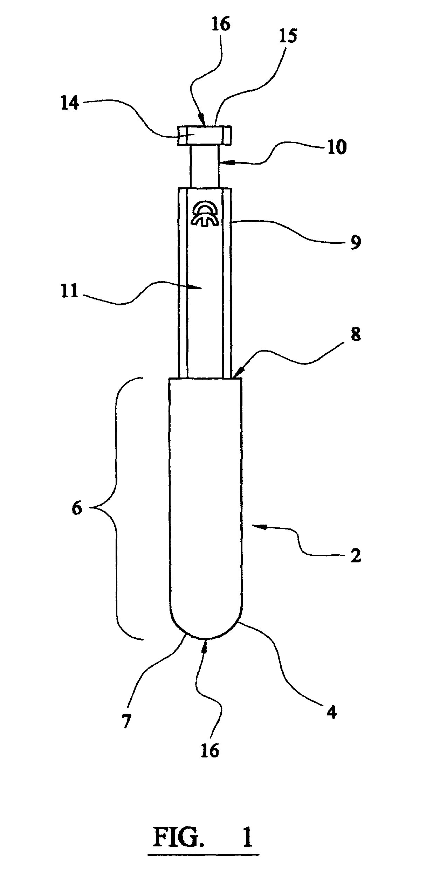

Referring to the drawings, Figure 1 shows a first tamp part 2 which has a tamp surface 4. The tamp surface extends over the entire distal portion 6 of the tamp part 2. The distal portion has a generally circular cross-section which is substantially constant over most of its length. The distal portion is generally rounded at its tip 7, and substantially planar at its end 8 which faces away from the tip 7. The tamp surface 4 is polished and free of defects.

The first tamp part 2 includes a shaft 9 which extends from the distal portion. The shaft has a generally circular cross-section and is smaller in diameter than the distal portion defined by the tamp surface 4. The shaft 9 has a flattened face 11 on one side.

The shaft has a circumferentially extending notch 10 formed in it towards its proximal end. A head 14 is provided between the notch and the proximal end 15. When viewed along the axis of the tamp part 2, the head is non-circular. Preferably, it has a flattened surface so that it is generally C-shaped when viewed along the axis.

The first tamp part has a locator bore 16 extending through its continuously between its ends. The bore can receive a guide wire which can be used to locate the tamp assembly.

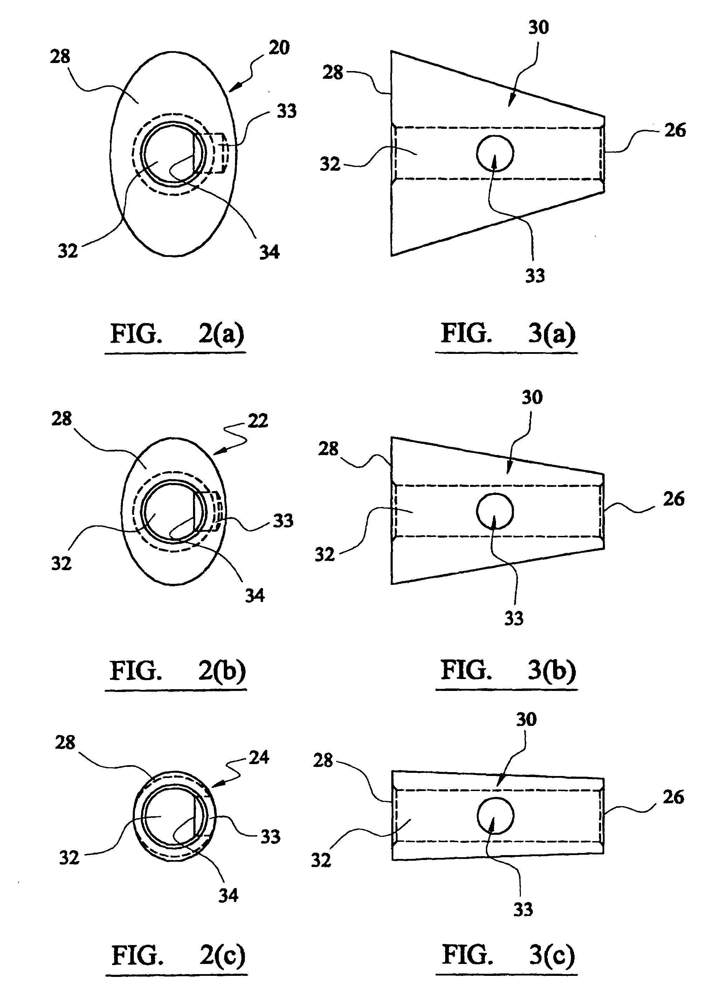

Figures 2 (a) to (c) show similar views of a set of second tamp parts 20, 22, 24 of which each one can be fitted with the first tamp part to form a useable tamp assembly. Each of the tamp parts is flared outwardly from its small end 26 which faces towards the first tamp part towards its big end 28 which faces towards the handle when the tamp is assembled. The cross-sections of the second tamp parts at the small ends 26 are substantially the same as the cross-section of the facing end 8 of the distal portion 6 of the first tamp part 2 at its end which faces away from the tip 7.

At their big ends 28, each of the second tamp parts has an elliptical cross-section in the sense that it is generally rounded but the transverse dimension on one axis is greater than that on a perpendicular axis. The ratio of the maximum transverse dimension on the major axis to the transverse dimension on the perpendicular minor axis is termed the eccentricity. The eccentricity of the cross-sections of the second tamp parts differs between the parts. The eccentricity is greatest for the second tamp part 20 that is shown in Figure 2 (a) and least for the second tamp part 24 shown in Figure 2 (c), the eccentricity ranging between about 1.7 and 1.1 across the three tamp parts. As can be seen from the drawings, the change in the cross-section of the second tamp parts between their small and big ends takes place gradually over their lengths. The external tamp surfaces 30 of the second tamp parts are polished and free of defects.

Each of the second tamp parts has a bore 32 extending through it in which the shaft 9 on the first tamp part 2 can be received. The cross-section of the bore 32 has a plug 33 extending into it with a flat internal face 34. The cross-section of the bore corresponds to that of the shaft 9 on the first tamp part (with the internal face 34 of the plug against the flat surface 11 of the shaft) so that the second tamp parts are a snug fit on the shaft. The plug acting against the flat surface of the shaft restricts relative rotation of the first and second tamp parts. The length of the second tamp parts (measured along the axis defined by the bore) is approximately equal to the length of the shaft 9, measured from the end 8 which faces away from the first tamp part tip 7 to the notch 10 in the shaft.

In a typical assembly, the dimensions of the components (in millimetres) might be as follows:

| First tamp part | |
|---|---|
| Length of distal portion | 60 |
| Diameter of distal portion at end 8 | 17 |
| Diameter of distal portion above tip 7 | 16 |
| Length of shaft to notch 10 | 45 |
| Length of notch 10 | 10 |
| Diameter of shaft | 12 |
| Diameter of through bore 16 | 3 |

| Second tamp parts | |
|---|---|
| Length | 45 |
| Diameter at small end | 17 |
| Max transverse dimension on large part | 30 |
| Max transverse dimension on small part | 16 |
| Min transverse dimension on large part | 18 |
| Min transverse dimension on small part | 14 |

The tamp of the invention is assembled by locating the shaft 9 on the first tamp part 2 within the bore 32 in one of the second tamp parts 20, 22, 24. The matched cross-sections of the distal portion of the first tamp part and the small end of the second tamp part provides a continuous tamping surface extending over the first and second parts. The head 14 is received in an opening 40 in a handle 42, which engages the shaft by means of a retractable tang which fits into the notch 10. The tang is operated by a sprung loaded collar 44 which can be actuated by sliding it axially along the handle.

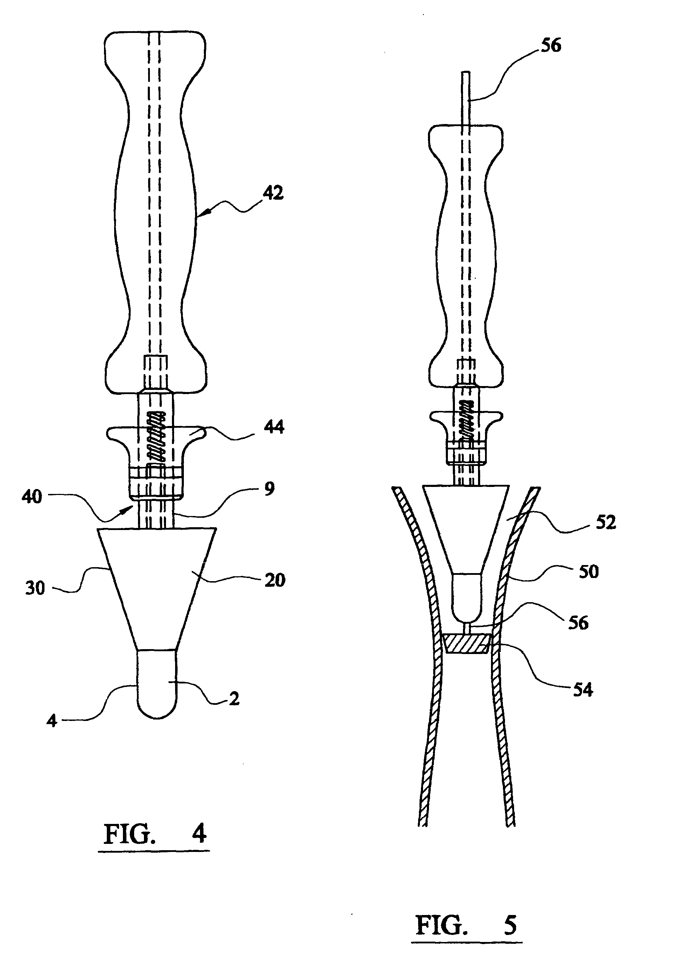

Figure 4 shows a patient's bone 50 (for example the tibia for replacement of the knee joint) which has been prepared to receive the tibial component of a knee joint prosthesis by resecting the bone and removing cancellous bone tissue from the medullary cavity 52. A bone plug 54 is then inserted into the cavity using known techniques and a guide wire 56 screwed into the bone plug. The cavity is then loaded with morcelised bone graft material which is intended to facilitate the growth of natural bone tissue in the region around an implanted prosthesis within the cavity, between the surface of the prosthesis and the internal surface of the cavity that is provided by the patient's cortical bone. Figure 4 shows the tamp assembly of the invention with the largest 20 of the three second tamp parts, located over the guide wire 56 so that it extends through the locator bore 16 in the first tamp part. The tamp can then be slid along the guide wire 56 so that the orientation of the tamp assembly is controlled as it forms the cavity in the morcelised bone graft within the cavity.

Once the bone graft material in the cavity has been compressed satisfactorily using the largest 20 of the second tamp parts, the tamp assembly is removed and more of the material is supplied to the cavity. The tamp is disassembled by actuating the lever on the handle and the largest of the second tamp parts is replaced by the intermediate second tamp part 22. The process is repeated until the bone graft material has been compressed using a tamp whose overall configuration matches that of the prosthesis part that is to be implanted.

## Claims

1. A tamp assembly for use in preparing a bone for implantation of a component of a joint prosthesis, comprising:
a. a handle (42),
b. a first tamp part (2) located remote from the handle, **characterised in that** the tamp assembly further comprises:
c. a plurality of second tamp parts (20,22,24), each of which can be located between the handle (42) and the first tamp part (2), the cross-sectional configuration of the second tamp parts (20,22,24) at their ends which face towards and contact the first tamp part (2) substantially matching the cross-sectional configuration of the first tamp part (2) on its end which faces and contacts the second tamp part (20,22,24), each of the second tamp parts (20,22,24) being flared outwardly from its end which faces towards the first tamp part (2) towards the end which faces towards the handle (42) with the shape of the flare varying between individual ones of the second tamp parts (20,22,24).

2. A tamp assembly as claimed in claim 1, in which the second tamp parts (20,22,24) have a cross-section at the end which faces towards the first tamp part (2) which is substantially circular, and a cross-section towards the end which faces towards the handle (42) which is substantially elliptical.

3. A tamp assembly as claimed in claim 2, in which the second tamp parts (20,22,24) differ from one another in the eccentricity of the elliptical cross-section at the end which faces the handle (42).

4. A tamp assembly as claimed in claim 3, in which the eccentricity of the elliptical cross-section of at least one of the second tamp parts (20,22,24) at the end which faces the handle is at least about 1.1.

5. A tamp assembly as claimed in claim 3, in which the eccentricity of the elliptical cross-section of at least one of the second tamp parts (20,22,24) at the end which faces the handle is not more than about 1.7.

6. A tamp assembly as claimed in claim 1, which includes at least three second tamp parts (20,22,24) which differ from one another in the shape of the flare between the end which faces towards the first tamp part (2) and the end which faces towards the handle (42).

7. A tamp assembly as claimed in claim 1, which includes a shaft (9) which extends between the handle (42) and the first tamp part (2) by which the handle (42) and the first and second tamp parts are connected to one another.

8. A tamp assembly as claimed in claim 7, in which the shaft (9) is attached to one of the handle (42) and the first tamp part (2), and can be separably connected to the other of the handle (42) and the first tamp part (2).

9. A tamp assembly as claimed in claim 1, which includes a guide wire (56) and in which the first and second tamp parts have a cannula extending through them in which the guide wire (56) can be received to control the orientation of the tamp parts when in use relative to the bone axis.

10. A tamp assembly as claimed in claim 9, which includes a plug (54) on which the guide wire (56) can be terminated.

## Patentansprüche

1. Stopfvorrichtung zur Präparation eines Knochens für eine Implantierung einer Komponente einer Gelenkprothese umfassend:
a. einen Handgriff (42),
b. ein erstes Stopfteil (2), das entfernt von dem Handgriff angeordnet ist, **dadurch gekennzeichnet, dass** die Stopfvorrichtung weiterhin umfasst:
c. eine Anzahl von zweiten Stopfteilen (20, 22, 24), die jeweils zwischen dem Handgriff (42) und dem ersten Stopfteil (2) angeordnet sein können, wobei die Querschnittskonfiguration der zweiten Stopfteile (20, 22, 24) an ihren Enden, die dem ersten Stopfteil (2) zugewandt sind und dieses berühren, im wesentlichen zu der Querschnittskonfiguration des ersten Stopfteiles (2) an seinem Ende, das dem zweiten Stopfteil (20, 22, 24) zugewandt ist und dieses berührt, passt, wobei jedes der zweiten Stopfteile (20, 22, 24) ausgehend von seinem Ende, das dem ersten Stopfteil (2) zugewandt ist, in Richtung des Endes, das dem Handgriff (42) zugewandt ist, nach außen erweitert ist, wobei die Form der Erweiterung zwischen verschiedenen zweiten Stopfteilen (20, 22, 24) variiert.

2. Stopfvorrichtung nach Anspruch 1, **dadurch gekennzeichnet, dass** die zweiten Stopfteile (20, 22, 24) einen Querschnitt, der im wesentlichen kreisförmig ist, an dem Ende aufweisen, das dem ersten Stopfteil (2) zugewandt ist, und dass die zweiten Stopfteile (20, 22, 24) einen Querschnitt, der im wesentlichen elliptisch ist, in Richtung des Endes aufweisen, das dem Handgriff (42) zugewandt ist.

3. Stopfvorrichtung nach Anspruch 2, **dadurch gekennzeichnet, dass** sich die zweiten Stopfteile (20, 22, 24) voneinander in der Exzentrizität des elliptischen Querschnittes an dem Ende, das dem Handgriff (42) zugewandt ist, unterscheiden.

4. Stopfvorrichtung nach Anspruch 3, **dadurch gekennzeichnet, dass** die Exzentrizität des elliptischen Querschnittes von zumindest einem der zweiten Stopfteile (20, 22, 24) an dem Ende, das dem Handgriff zugewandt ist, zumindest ungefähr 1.1 beträgt.

5. Stopfvorrichtung nach Anspruch 3, **dadurch gekennzeichnet, dass** die Exzentrizität des elliptischen Querschnittes von zumindest einem der zweiten Stopfteile (20, 22, 24) an dem Ende, das dem Handgriff zugewandt ist, nicht mehr als ungefähr 1.7 beträgt.

6. Stopfvorrichtung nach Anspruch 1, **dadurch gekennzeichnet, dass** die Stopfvorrichtung zumindest drei zweite Stopfteile (20, 22, 24) umfasst, die sich voneinander in der Form der Erweiterung zwischen dem Ende, das dem ersten Stopfteil (2) zugewandt ist, und dem Ende, das dem Handgriff (42) zugewandt ist, unterscheiden.

7. Stopfvorrichtung nach Anspruch 1, **dadurch gekennzeichnet, dass** die Stopfvorrichtung einen Schaft (9) umfasst, der sich zwischen dem Handgriff (42) und dem ersten Stopfteil (2) erstreckt, durch den der Handgriff (42) und der erste und die zweiten Stopfteile miteinander verbunden sind.

8. Stopfvorrichtung nach Anspruch 7, **dadurch gekennzeichnet, dass** der Schaft (9) entweder an dem Handgriff (42) oder an dem ersten Stopfteil (2) befestigt ist und dass der Schaft abnehmbar mit dem anderen Teil, Handgriff (42) oder erstem Stopfteil (2), verbunden ist.

9. Stopfvorrichtung nach Anspruch 1, **dadurch gekennzeichnet, dass** die Stopfvorrichtung einen Führungsdraht (56) aufweist und dass das erste und die zweiten Stopfteile eine Röhre aufweisen, die sich durch sie erstreckt und in der der Führungsdraht aufgenommen werden kann, um die Orientierung der Stopfteile relativ zu der Knochenachse im Gebrauch zu kontrollieren.

10. Stopfvorrichtung nach Anspruch 9, **dadurch gekennzeichnet, dass** die Stopfvorrichtung einen Stopfen (54) aufweist, durch den der Führungsdraht (56) abgeschlossen sein kann.

## Revendications

1. Ensemble de tassage à utiliser pour préparer un os pour l'implantation d'un composant de prothèse articulaire, comprenant :
a. une poignée (42)
b. une première partie de tassage (2) située à distance de la poignée, **caractérisé en ce que** l'ensemble de tassage comprend en outre :
c. une pluralité de secondes parties de tassage (20, 22, 24) dont chacune peut être située entre la poignée (42) et la première partie de tassage (2), la configuration en coupe des secondes parties de tassage (20, 22, 24) au niveau de leur extrémité qui fait face à et qui est en contact avec la première partie de tassage (2) correspondant sensiblement à la configuration de la section transversale de la première partie de tassage (2) sur son extrémité qui fait face à et est en contact avec la seconde partie de tassage (20, 22, 24), chacune des secondes parties de tassage (20, 22, 24) étant élargie vers l'extérieur depuis son extrémité qui fait face à la première partie de tassage (2) vers l'extrémité qui fait face à la poignée (42), la forme de l'élargissement variant selon chaque seconde partie de tassage (20, 22, 24).

2. Ensemble de tassage selon la revendication 1, dans lequel les secondes parties de tassage (20, 22, 24) ont une section transversale au niveau de l'extrémité qui fait face à la première partie de tassage (2) qui est sensiblement circulaire et une section transversale vers l'extrémité qui fait face à la poignée (42) qui est sensiblement elliptique.

3. Ensemble de tassage selon la revendication 2, dans lequel les secondes parties de tassage (20, 22, 24) diffèrent les unes des autres par l'excentricité de la section transversale elliptique au niveau de l'extrémité qui fait face à la poignée (42).

4. Ensemble de tassage selon la revendication 3, dans lequel l'excentricité de la section transversale elliptique d'au moins une des secondes parties de tassage (20, 22, 24) au niveau de l'extrémité qui fait face à la poignée est d'au moins environ 1,1.

5. Ensemble de tassage selon la revendication 3, dans lequel l'excentricité de la section transversale elliptique d'au moins une des secondes parties de tassage (20, 22, 24) au niveau de l'extrémité qui fait face à la poignée est d'au moins environ 1,7.

6. Ensemble de tassage selon la revendication 1, qui inclut au moins trois secondes parties de tassage (20, 22, 24) qui diffèrent les unes des autres par la forme de l'élargissement entre l'extrémité qui fait face à la première partie de tassage (2) et l'extrémité qui fait face à la poignée (42).

7. Ensemble de tassage selon la revendication 1, qui inclut une tige (9) qui s'étend entre la poignée (42) et la première partie de tassage (2) par laquelle la poignée (42) et les première et seconde parties de tassage sont raccordées.

8. Ensemble de tassage selon la revendication 7, dans lequel la tige (9) est fixée à l'une de la poignée (42) et de la première partie de tassage (2) et peut être raccordée de manière séparable à l'autre de la poignée (42) et de la première partie de tassage (2).

9. Ensemble de tassage selon la revendication 1, qui inclut un fil guide (56) et dans lequel les première et seconde parties de tassage ont une canule qui les traverse dans laquelle le fil guide (56) peut être placé pour contrôler l'orientation des parties de tassage par rapport à l'axe de l'os lors de leur utilisation.

10. Ensemble de tassage selon la revendication 9, qui inclut un bouchon (54) sur lequel le fil guide (56) peut se terminer.
